# EUROPEAN PATENT SPECIFICATION

(11) **EP 1 600 172 B1**
(45) Date of publication and mention of the grant of the patent: **26.11.2008**
(21) Application number: 05009883.9
(22) Date of filing: 06.05.2005
(51) Int. Cl.: A61L 2/12

(54) **Device for disinfesting and drying wooden packaging material**
Vorrichtung zur Entwesung und Trocknung von Verpackungsmaterialien aus Holz
Dispositif pour désinfester et sécher des emballages en bois

(30) Priority: 26.05.2004 IT MI20041053
(43) Date of publication of application: 30.11.2005
(73) Proprietor: EMITECH S.R.L., 70033 Corato (BA) (IT)
(72) Inventor: Diaferia, Nicola, 70033 Corato (BA) (IT)
(74) Representative: Petruzziello, Aldo

(56) References cited:
- WO-A-02/14764
- DE-U1- 9 413 736
- GB-A- 2 166 633
- GB-A- 2 292 084
- US-A1- 2003 057 203
- DATABASE WPI Section Ch, Week 199315 Derwent Publications Ltd., London, GB; Class D22, AN 1993-124532 XP002334909 "Wood packaging sterilisation method - by treating with nitrogen di:oxide and simultaneously exposing to microwave radiation to raise reliability" -& SU 1 729 514 A 30 April 1993 (1993-04-30)

## Description

The present invention has as its field of application the wooden packaging sector and refers in general to a device and a method for disinfestation and drying of wooden packaging materials.

The term "wooden packaging" should include not only finished products such as pallets, cases, etc. but also semi-finished products such as boards, wedges, etc., used to make the above and also raw wood before it is subjected to sawing.

Wooden packaging material constitutes an excellent passive means of spreading the most formidable phytophagous organisms (xylophagous insects, nematodes and other pests typical of such materials). The Food and Agriculture Organization (FAO) is sensitive to the problem and has issued directive ISPM15 containing measures such as to limit the spread of such organisms with international trade.

The measures currently envisaged are:
a) Fumigation with methyl bromide (MB) with T > 10°C, tₑₓₚₒₛᵤᵣₑ>16 h
b) Heat treatment (HT) with T = 56 °C, tₑₓₚₒₛᵤᵣₑ = 30'

These measures present drawbacks. The use of a fumigant like methyl bromide is recognised as highly dangerous by the Montreal Protocol. Heat treatment has a lower impact on the environment. However, it presents limitations due to expenditure in terms of time for treatment and power consumption that represent an obstacle to the practical feasibility of the method.

On the other hand, the time/temperature combination established for heat treatment (HT) proves to be a sort of compromise between a relatively acceptable efficacy (although it is known that some noxious organisms withstand the established temperature) and commercially sustainable costs.

WO 02/14764 discloses a process ad apparatus for drying and/or pathogen reduction by means of microwaves.

GB 2 166 633 discloses a process an apparatus for sterilisation of horticultural material by means of microwaves.

GB 2 292 084 discloses a microwave pest exterminator suitable for timber and wood furniture.

SU 1 729 514 disclose a wood packaging sterilisation method by treating with nitrogen dioxide and simultaneously exposing to microwave radiation.

The object of the present invention is to eliminate the drawbacks of the prior art by providing a device and a method for disinfesting and drying wooden packaging materials that is efficient, rapid and economical.

Another object of the present invention is to provide a device and a method for disinfesting and drying wooden packaging materials that is safe for the operator and the environment.

These objects are achieved in accordance with the invention with the characteristics - described in the device and in the method according to claim 1.

Advantageous embodiments of the invention are apparent from the dependent claims.

The invention stems from the observation that many infesting biological forms do not survive beyond a certain temperature called the "lethal temperature" (LT). Exploiting the thermal effects of microwaves related to the interaction of magnetic fields with polar molecules (particularly water) or charged particles (ions), it is possible to raise the body temperature of the infesting biological forms above their LT.

Therefore, the microwave apparatus according to the invention is able to induce a temperature distribution within the infesting agents such as to ensure that:
- biological pests are eliminated once they are brought to temperatures above those that are lethal to them;
- the temperature of the material subject to treatment is not such as to cause significant mechanical and/or physical stresses in the material.

The different thermal behaviours of the pests and the material subjected to treatment is related to the different dielectric characteristics thereof and in particular to the greater percentages of water contained in the pests which, therefore, will reach considerably higher temperatures with respect to those detected in the materials treated.

The microwave treatment according to the invention presents the following advantages:
- High disinfesting efficacy on xylophagous insects in any life stage (adult insect, larva, pupa, egg), on nematodes and on other species of pests, such as chromogenic fungi which are responsible for the colourings of wood (characteristic of the individual types of fungus in the interaction with the host plant).
- Attainment of a temperature considerably higher than 56°C (thus extending the application to groups of quarantine organisms resistant to this temperature).
- Extremely low treatment time. In a few minutes of radiation, temperatures near 90°C are reached and maintained without any further energy input. Furthermore, because of the peculiar thermal effect induced by microwaves, no transition times are necessary to reach the disinfestation temperature: treatment times coincide with disinfestation times.
- Significant energy saving with respect to conventional heat methods. In fact, the energy radiated in the system according to the invention is absorbed exclusively by the dielectric material treated (wood) and more than 90% of the energy irradiated by the microwave generators and reverberated by the electromagnetic stirrers is converted into heat.
- Complete absence of electromagnetic pollution of the operating environment, since the electromagnetic energy irradiated is entirely confined inside the microwave-shielded reverberation structure. Thus, protection of the operators is totally ensured.
- Just in time treatment: microwave treatment does not require time for activation of the system like conventional treatments; therefore the treatment time coincides with the time for the disinfestation/drying process.

All these advantages derive from the particular mode of action of microwaves, which allow centrifugal heating. In fact, microwaves have a high depth of penetration into the wood with consequent induction of the thermal process within the material treated. The low heat conductivity of wood means that the core of the wood reaches and maintains stable high temperatures for the times necessary to ensure full disinfesting efficacy. As a result thermal uniformity of the treatment is obtained through the uniformity of the electromagnetic irradiation.

From the experiments conducted it is deduced that the most difficult parts of the packaging to treat with conventional HT heat treatment techniques are advantageously treated with microwaves. It has also been demonstrated experimentally that the points of contact between boards, wedges etc., critical areas for infestation with pests, reach the maximum temperatures (hot spots) during microwave treatment.

Furthermore with the microwave treatment according to the invention considerable drying of the wood is achieved, the packaging materials are kept intact even at temperatures near to 85°C and near metal parts (nails, screws, etc.), and movement of the packaging is possible immediately after disinfestation, since the electromagnetic energy does not persist at the end of treatment.

The microwave treatment according to the invention can also be used for quarantine procedures on wooden packaging materials.

Further characteristics of the invention will be made clearer by the detailed description that follows, referring to purely exemplary and therefore non-limiting embodiments thereof, in which:
Figure 1 is a diagrammatic side elevation view, partially broken off and in axial section, illustrating a first embodiment of a device for disinfestation and drying of wooden packaging materials;
Figure 2 is a cross sectional view taken along the plane of section II-II in Figure 1;
Figure 3 is a diagrammatic top plan view diagrammatically illustrating the device of Figure 1 inserted in a production line for wooden packaging materials;
Figure 4 is a top plan view illustrating a second embodiment of a device for disinfestation and drying of wooden packaging materials;
Figure 5 is a cross sectional view taken along the plane of section V-V of Figure 4;

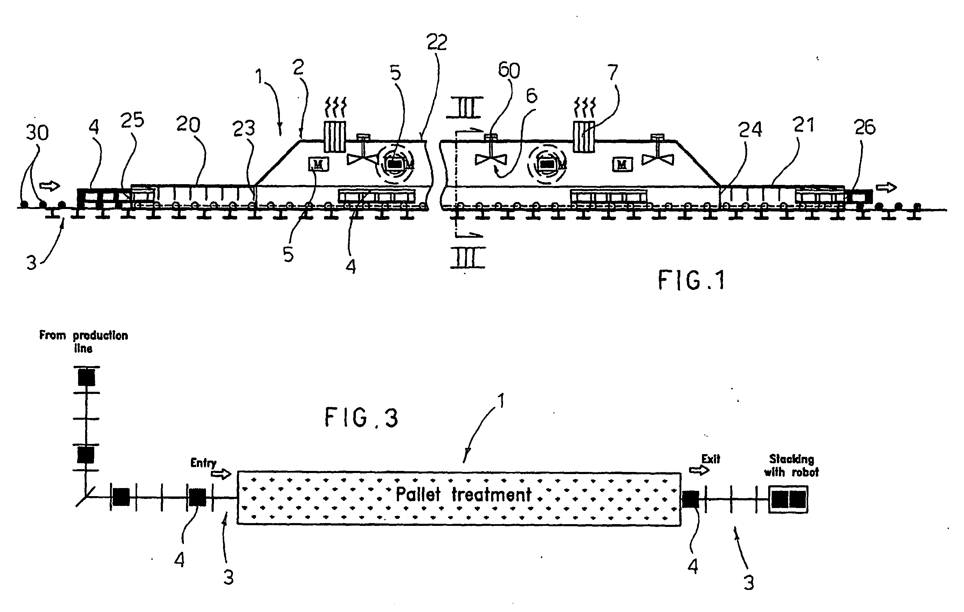

With reference for now to Figures 1 - 3 a device for disinfesting and drying wooden packaging materials according to a first embodiment, indicated as a whole with reference numeral 1 is described.

The device 1 comprises a tunnel 2 shielded to microwaves. The tunnel 2 comprises an entry connecting part 20, an exit connecting part 21 and a central operating part 22. A conveyor 3 able to convey wooden packaging material, such as pallets 4, for example, passes inside the tunnel 2.

The conveyor 3 is a motorised roller train conveyor belt 30 of the type already used in pallet production lines. In this manner the production line is not modified, but its terminal part is simply lengthened.

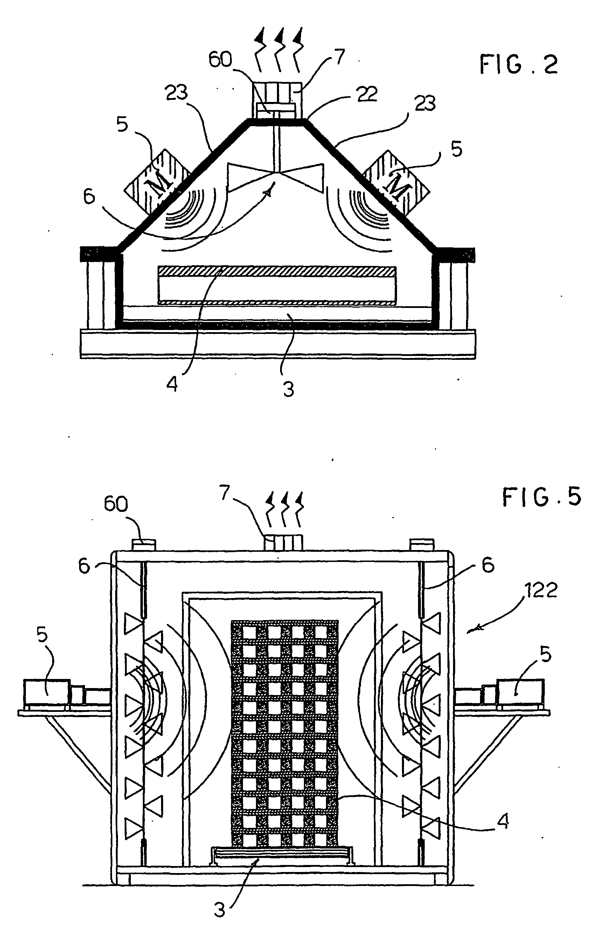

The entry and exit connecting parts 20 and 21 of the tunnel 2 have a substantially parallelepiped shape with a rectangular section able to allow the passage of one pallet 4 at a time, conveyed on the conveyor 3. As also shown in Figure 2, the central operating part 22 of the tunnel 2 is longer and higher than the connecting parts 20, 21. The central operating part 22 is pentagonal in cross section and has two converging sloping side walls 23, with an angle of about 45° with respect to a horizontal plane, which extend above the height of the pallet 4 placed on the conveyor 3.

Microwave generators (magnetrons) 5 are installed on the sloping side walls 23. The magnetrons 5 are disposed outside the tunnel 2 and in register with the openings in the side walls 23 so as to irradiate electromagnetic waves at microwave frequency (2.45 GHz) inside the central part 22 of the tunnel, which thus behaves as a microwave reverberating room. By way of example, magnetrons 5 with a power of about 6 kW can be used.

On the roof of the central operating part 22 of the tunnel at least one electromagnetic mode stirrer 6 is installed. The stirrer 6 consists of metal blades with a suitable geometry, and it is disposed inside the reverberation chamber so as to be situated in a central position above the pallets 4 conveyed by the conveyor 3. The stirrer 6 is operated in rotation by an electric motor 60 disposed on the roof of the central operating part 22 outside the reverberation chamber.

Also on the roof of the central operating part 22, air inlets 7, equipped with an electromagnetic filter, are provided for suction from the reverberating chamber of the vapour obtained from treatment of the pallets 4.

The tunnel 2 can be of the modular type with shielded modules connected to each other electrically through mechanical joining elements and clamping obtained with bolting of a suitable pitch. Each module of the central operating part 22 is provided with one or more microwave power generators 5, with a stirrer system (electromagnetic mode stirrers) 6 and a moisture suction and evacuation system 7, provided with an electromagnetic filter. The tunnel 2, being made in modules, can be of variable length.

The dimensions of the cross section of the tunnel 2 have been designed to optimise the electromagnetic reverberation process and allow treatment of pallets made according to EPAL standards, such as Euro-Pallets.

At the entry and exit of the central operating part 22 there are automated shielded gates 23, 24, designed to ensure the best electromagnetic uncoupling between the outside environment and the treatment chamber and allow transfer of the pallets 4 into the central operating part 22. Control of opening and closing of the automated gates 23, 24 is obtained by means of dedicated logic.

At the entry and exit of the treatment chamber defined by the central operating part 22, there are respective shielded connecting elements 20 and 21 which act as a connection with the outside environment.

The shielded connecting elements 20 and 21 are always electrically connected to the central operating part 22. These connecting elements 20, 21 are also provided with automated shielded gates 25 e 26, controlled through dedicated logic, which must close when the gates 23, 24 of the treatment chamber open, for entry and exit of the pallets 4. In this manner the homogeneity of the electromagnetic shield is always ensured and the irradiation process can be continuative, eliminating idle times due to loading/unloading of the pallets 4 into/from the treatment chamber 22.

As shown in Figure 3, the device 1 according to the first embodiment of the invention is particularly suitable for large industrial pallet production plants and allows continuous treatment within the pallet production line. The tunnel 2 can be installed at the end of the pallet production line and immediately before the automatic system (robot) for stacking of pallets 4.

This type of system allows the disinfestation/drying treatment times to be adjusted by means of the device 1 to the production times (180-360 pallets/h) of the plants that will house the device 1 without slowing their production. This is achieved whilst still guaranteeing the primary purpose of the process, which is complete phytosanitary efficacy.

For this purpose the following system parameters can be set up: the appropriate length of the treatment 22, the exposure times of the pallets 4 and the electromagnetic powers irradiated by the magnetrons 5. Purely by way of example, the treatment chamber 22 may have a length between 15 m and 20 m, the exposure time of the pallets 4 inside the treatment chamber 22 is about 60-120 seconds, and magnetrons 5 with a power of 6 kW are used.

If necessary, the device 1 can be multiplied on more than one treatment line, in the case of very fast production lines (more than 360 pallets/h) or if the logistic requirements of the plant do not allow substantial lengths of the treatment tunnel 2.

The device 1 is provided at the entry and exit with sensors for thermohygrometric testing of the material before and after treatment, in order to achieve better calibration of the microwave heat process (also achieved through a feedback on the microwave generators) and effective control downstream of the thermohygrometric conditions of the irradiated material.

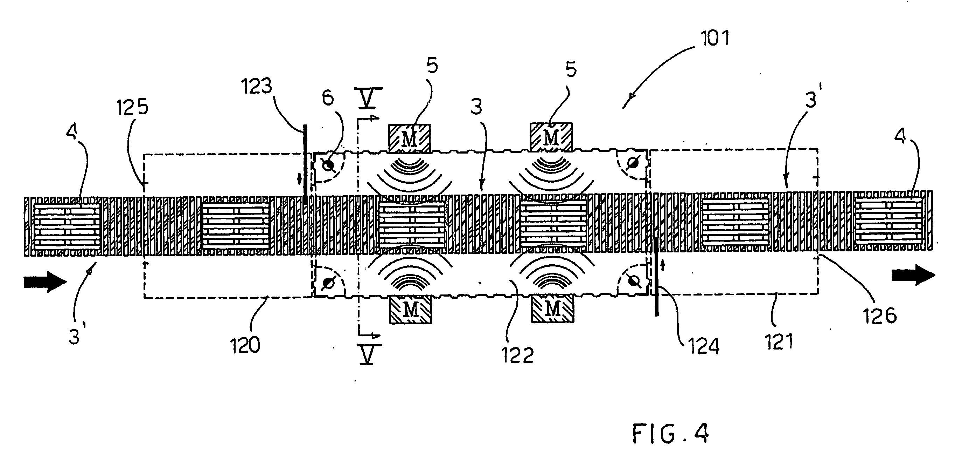

With reference to Figures 4 and 5 a disinfesting and drying device 101 for wooden packaging materials according to a second embodiment of the invention is described, in which like or corresponding elements to those already described are indicated with the same reference numerals and detailed description thereof is omitted.

The device 101 comprises a microwave shielded reverberating cell 122 for disinfestation/drying of stacks of wooden packaging material, finished (pallets) 4 and/or semi-finished products (boards, wedges, etc.) and/or raw wood.

This design solution is particularly suitable for small and medium sized wooden packaging production companies and repair companies. Moreover it can certainly also be adopted by companies using wooden packaging (for commercial purposes) tied to strict quality constraints on the quality of the products distributed, such as large distribution companies in the agro-feeing, pharmaceutical, and textile sectors, etc.

For small and medium wooden packaging production companies and repair companies it is not mandatory to have disinfesting/drying treatments in the production line. In fact, for these companies it is essential to perform disinfesting/drying treatment in a flexible manner, adjusting to the production requirements of the particular moment and to the working speed, and carrying out just-in-time treatment on large volumes of material, perhaps already stacked and ready for despatch. Furthermore, repair companies have much higher working times than production companies, therefore inserting a very fast treatment on a slow processing line would not be advantageous. In fact for these companies it is important instead to use the treatment system 101 on material present in the warehouse.

The treatment device 101 comprises a microwave shielded reverberating chamber 122 made for example in a substantially parallelepiped shaped ISO standard container. Several microwave power generation apparatuses (magnetrons) 5 are installed on the side walls of the shielded chamber 122, their number being planned according the load requirements of the system.

At the four corners and/or along the walls of the shielded chamber 122 various electromagnetic mode stirrer apparatuses 6 are disposed. Each stirrer 6 has a plurality of blades disposed radially on a shaft that extends from the floor to the roof of the chamber 122. The means of driving the stirrer 6 are situated on the outside (for example on the roof) of the chamber 122 and can comprise an electric motor 60 that directly rotates the shaft of the stirrer 6 either through cams or a linear actuator or the cylinder-piston type that sets the stirrer 6 in vibration.

The position of the stirrers 6 and of the microwave irradiating mouths 5 is designed to obtain optimisation of the uniformity and homogeneity of irradiation of the treated material 4 and thus of the heating process induced by the microwaves on the treated material 4.

The treatment chamber 122 is provided with a shielded entry door 123 and a shielded exit door 124, both automated, and an internal conveying system, for example on a motorised roller bed. In this manner, it is possible to load and unload the treatment chamber 122 automatically by means of a dedicated control logic that manages the opening/closing times of the doors 123, 124 and advancement of the conveyor 3. Loading and unloading is also facilitated by the presence of respective transfer means 3', for example on motorized roller beds, downstream and upstream of the treatment chamber 3. When the doors 123, 124 are open the outside roller beds 3' are adjacent to the inside roller bed 3 allowing transfer of the material 4 from the outside to the inside of the chamber 122 and vice versa, without the aid of auxiliary conveying means or operating units.

This type of system, because of the way it is configured, allows stacks of finished material (pallets), large volumes of semi-finished material (boards, wedges, etc,) or raw wood to be treated in very short times.

The system is provided with a entry connecting part as pre-chamber 120 and an an exit connecting part or after-chamber 121, respectively downstream and upstream of the treatment chamber 122. The pre-chamber 120 and after-chamber 121 are also shielded to radio frequencies and form a homogeneous electromagnetic shield with the treatment chamber 122. The two chambers 120, 121 are provided on their inside with a motorized roller bed 3', so as to create a continuity between the external conveying apparatus and that inside the treatment chamber 122.

The two chambers 120, 121 are smaller in size than the treatment chamber 122; the pre-chamber 120 is provided with an automated shielded entry 125 and the after-chamber with an exit 126 of the same type.

Automated management of the entries and exits of the three chambers 120, 122, 121 allows operation of the accesses similar to that of the tunnel system 2 of the first embodiment described above to e achieved. The outermost accesses 125, 126 must close when the accesses 123, 124 of the treatment chamber open for entry or exit of the stacks of pallets 4 or of semi-finished or raw material. In this manner the homogeneity of the electromagnetic field is always ensured and the irradiation process can be continuative, eliminating idle times related to loading/unloading of the treatment chamber 122.

Furthermore, using the pre-chamber 120 and the after-chamber 121 it is certainly easier to implement automatisms for thermohygrometric testing of the material before and after treatment; this serves to achieve better calibration of the microwave thermal process (also achieved through a feedback on the microwave generators) and an effective downstream control of the thermohygrometric conditions of the irradiated material.

Numerous changes or modifications of detail within the reach of a person skilled in the art can be made to the present embodiments of the invention without departing from the scope of the invention as set forth in the appended claims.

## Claims

1. A device (1; 101) for disinfesting and drying wooden packaging material (4) comprising:
- a reverberating chamber (22; 122) shielded to radio frequencies,
- at least one microwave generator or magnetron (5) able to radiate microwaves inside said reverberating chamber (22; 122), and
- conveying means (3, 3', 30) able to convey said wooden packaging materials (4) into said reverberating chamber (22; 122) in which they are treated with microwaves until the lethal temperature for biological pests is reached and evaporation of moisture from the wood is achieved to obtain drying,
- a shield entry connecting part (20; 120), a shield exit connecting part (21; 121) connected respectively upstream and downstream of said reverberating chamber (22; 122) to form a homogeneous electromagnetic shield with the reverberating chamber (22; 122),
**characterised in that**
said conveying means (3, 3', 30) are a roller bed conveyor (30) and
said reverberating chamber (22; 122) comprises a shielded entry door (23; 123) and a shielded exit door (24; 124) and said entry connecting part (20; 120) and exit connecting part (21, 121) comprise respectively a shielded entry door (25; 125) and a shielded exit door (26; 126) which open/close alternately with respect to said entry (23; 126) and exit doors (24; 124) of the reverberating chamber (22; 122), so that the microwave irradiation process can be continuative, constantly guaranteeing the homogeneity of the electromagnetic shield.

2. A device according to claim 1, **characterised in that** inside said reverberating chamber (22; 122) there is inserted at least one stirrer (6) able to mix the electromagnetic modes at the microwave frequency to obtain a uniform distribution of the electromagnetic field within the reverberating chamber (22; 122).

3. A device according to claim 1 or 2, **characterised in that** said reverberating chamber (22; 122) is provided with at least one air inlet (7) provided with an electromagnetic filter, for aspiration and evacuation of the vapour generated in said reverberating chamber (22; 122) for treatment of wooden packaging materials (4).

4. A device (1) according to any one of the preceding claims, **characterised in that** it is inserted in a production line for wooden packaging materials (4) and that said roller bed conveyor (30) is able to convey said wooden materials continuously, at the production speed, into the reverberating chamber (22).

5. A device (1) according to any one of claims 1 to 4, **characterised in that** said reverberating chamber (22) is of the modular type and is formed by a plurality of modules connected so as to obtain the desired length, in which each module is provided with microwave generating means (5).

6. A device (1; 101) according to any one of the preceding claims, **characterised in that** it comprises temperature and humidity sensors able to perform a thermohygrometric test on the wooden materials (4) treated inside said reverberating chamber (22; 122).

## Patentansprüche

1. Eine Vorrichtung (1; 101) zur Entwesung und zum Trocknen von Verpackungsmaterialien (4), Folgendes umfassend:
- eine Reflexionskammer (22; 122), die gegen Funkfrequenzen abgeschirmt ist,
- mindestens einen Mikrowellengenerator oder Magnetron (5), der Mikrowellen innerhalb der Reflexionskammer (22; 122) abstrahlen kann, und
- Fördermittel (3, 3', 30), die die Verpackungsmaterialien aus Holz (4) in die Reflexionskammer (22; 122) befördern können, in welcher sie mit Mikrowellen behandelt werden, bis die tödliche Temperatur für biologisches Ungeziefer erreicht ist und das Verdampfen von Feuchtigkeit aus dem Holz verwirklicht wird, um das Trocknen zu erzielen,
- einen Abschirmungseingangsverbindungsteil (20; 120), einen Abschirmungsausgangsverbindungsteil (21; 121), die jeweils stromaufwärts und stromabwärts von der Reflexionskammer (22; 122) angeschlossen sind, um eine homogene elektromagnetische Abschirmung mit der Reflexionskammer (22; 122) zu bilden,
**dadurch gekennzeichnet,**
**dass** die Fördermittel (3, 3', 30) ein Rollengangförderer (30) sind, und
**dass** die Reflexionskammer (22; 122) eine abgeschirmte Eingangstür (23; 123) und eine abgeschirmte Ausgangstür (24; 124) aufweist, und dass der Eingangsverbindungsteil (20; 120) und der Ausgangsverbindungsteil (21; 121) jeweils eine abgeschirmte Eingangstür (25; 125) und eine abgeschirmte Ausgangstür (26; 126) aufweisen, die sich abwechselnd in Bezug zu der Eingangstür (23; 126) und der Ausgangstür (24; 124) der Reflexionskammer (22; 122) öffnen/schließen, so dass der Mikrowellenstrahlungsprozess kontinuierlich sein kann, um die Homogenität der elektromagnetischen Abschirmung ständig zu garantieren.

2. Vorrichtung nach Anspruch 1, **dadurch gekennzeichnet, dass** in das Innere der Reflexionskammer (22; 122) mindestens ein Rührwerk (6) eingefügt ist, das die elektromagnetischen Modi bei der Mikrowellenfrequenz mischen kann, um eine gleichförmige Verteilung des elektromagnetischen Felds in der Reflexionskammer (22; 122) zu erzielen.

3. Vorrichtung nach Anspruch 1 oder 2, **dadurch gekennzeichnet, dass** die Reflexionskammer (22; 122) mit mindestens einem Lufteinlass (7) versehen ist, der mit einem elektromagnetischen Filter zum Ansaugen und Ableiten des Dampfs, der in der Reflexionskammer (22; 122) zur Behandlung der Verpackungsmaterialien aus Holz (4) erzeugt wird, versehen ist.

4. Vorrichtung (1) nach einem der vorhergehenden Ansprüche, **dadurch gekennzeichnet, dass** sie in eine Produktionslinie für Verpackungsmaterialien aus Holz (4) eingefügt ist, und dass der Rollengangförderer (30) die Materialien aus Holz kontinuierlich mit Produktionsgeschwindigkeit in die Reflexionskammer (22) befördern kann.

5. Vorrichtung (1) nach einem der Ansprüche 1 bis 4, **dadurch gekennzeichnet, dass** die Reflexionskammer (22) modular ist und aus mehreren Modulen gebildet ist, die derart verbunden sind, dass die erwünschte Länge, bei der jedes Modul mit Mikrowellenerzeugungsmitteln (5) versehen ist, erzielt wird.

6. Vorrichtung (1; 101) nach einem der vorhergehenden Ansprüche, **dadurch gekennzeichnet, dass** sie Temperatur- und Feuchtigkeitssensoren aufweist, die an den Materialien aus Holz (4), die in der Reflexionskammer (22; 122) behandelt werden, einen thermohygrometrischen Test ausführen können.

## Revendications

1. Dispositif (1; 101) pour désinfecter et sécher des emballages en bois (4), comprenant :
- une chambre de réverbération (22 ; 122) blindée par un écran contre les radiofréquences,
- au moins un générateur de micro-ondes ou magnétron (5) apte à rayonner des micro-ondes à l'intérieur de ladite chambre de réverbération (22 ; 122), et
- des moyens de transport (3, 3', 30) aptes à transporter lesdits emballages en bois (4) dans ladite chambre de réverbération (22 ; 122) dans laquelle ils sont traités avec des micro-ondes jusqu'à ce que la température létale pour des parasites biologiques soit atteinte et l'évaporation de l'humidité du bois soit réalisée afin d'obtenir le séchage,
- une partie de raccord d'entrée d'écran (20 ; 120), une partie de raccord de sortie d'écran (21 ; 121) raccordés respectivement en amont et en aval de ladite chambre de réverbération (22 ; 122) afin de former un écran électromagnétique homogène avec la chambre de réverbération (22 ; 122),
**caractérisé en ce que**
lesdits moyens de transport (3, 3', 30) sont un transporteur à rouleaux (30) et
ladite chambre de réverbération (22 ; 122) comprend une porte d'entrée blindée par un écran (23 ; 123) et une porte de sortie blindée par un écran (24 ; 124) et ladite partie de raccord d'entrée (20 ; 120) et ladite partie de raccord de sortie (21 ; 121) comprennent respectivement une porte d'entrée blindée par un écran (25 ; 125) et une porte de sortie blindée par un écran (26 ; 126) qui s'ouvrent/se ferment en alternance par rapport à ladite porte d'entrée (23 ; 126) et ladite porte de sortie (24 ; 124) de la chambre de réverbération (22 ; 122), de telle sorte que le processus d'irradiation aux micro-ondes puisse être continu, en garantissant constamment l'homogénéité du champ électromagnétique.

2. Dispositif selon la revendication 1, **caractérisé en ce que** à l'intérieur de ladite chambre de réverbération (22 ; 122) est inséré au moins un mélangeur (6) apte à mélanger les modes électromagnétiques à la fréquence micro-onde afin d'obtenir une distribution uniforme du champ électromagnétique à l'intérieur de la chambre de réverbération (22 ; 122).

3. Dispositif selon la revendication 1 ou 2, **caractérisé en ce que** ladite chambre de réverbération (22 ; 122) est pourvue d'au moins une admission d'air (7) pourvue d'un filtre électromagnétique, pour l'aspiration et l'évacuation de la vapeur générée dans ladite chambre de réverbération (22 ; 122) à des fins de traitement des emballages en bois (4).

4. Dispositif (1) selon une quelconque des revendications précédentes, **caractérisé en ce que** il est inséré dans une ligne de production d'emballages en bois (4) et que ledit transporteur à rouleaux (30) est apte à transporter en continu lesdits matériau de bois, à la vitesse de production, dans la chambre de réverbération (22).

5. Dispositif (1) selon une quelconque des revendications 1 à 4, **caractérisé en ce que** ladite chambre de réverbération (22) est du type modulaire et est formée par une pluralité de modules raccordés de manière à obtenir la longueur désirée, à laquelle chaque module est pourvu de moyens de génération de micro-ondes (5).

6. Dispositif (1 ; 101) selon une quelconque des revendications précédentes, **caractérisé en ce que** il comprend des capteurs de température et d'humidité aptes à effectuer un test thermohygrométrique sur les matériaux de bois (4) traités à l'intérieur de ladite chambre de réverbération (22 ; 122).
